# EUROPEAN PATENT APPLICATION

(11) **EP 4 548 803 A1**
(43) Date of publication of application: **07.05.2025**
(21) Application number: 23207538.2
(22) Date of filing: 02.11.2023
(51) Int. Cl.: A45D 26/00, A61N 5/06

(54) **PERSONAL CARE APPLIANCE**

(71) Applicant: Koninklijke Philips N.V., 5656 AG Eindhoven (NL)
(72) Inventor: DE GOEIJ, Geert Willem, 5656 AG Eindhoven (NL); DE GROOT, Ronald, 5656 AG Eindhoven (NL)
(74) Representative: Philips Intellectual Property & Standards

(57) **Abstract**

The subject-matter of the present disclosure relates to a personal care appliance (100). The personal care appliance (100) comprises: a handle (102); a first switch (300) on the handle; a second switch on the handle; an illumination device (520); a detection module (114) configured to detect when at least one of a first operational head (106) is attached to the handle, a second operational head (120) is attached to the handle, and an attachment (107) is attached to the first operational head; and a controller (110) configured to control a primary function of the first or second operational head in response to actuation of the first switch when the first or second operational head are attached to the handle, to activate the illumination device in response to the detection module detecting the second operational head is attached to the handle or the attachment to the first operational head is attached to the first operational head, and, in response to actuation of the second switch, control a secondary function of the second operational head when the second operational head is attached to the handle, and control a function of the attachment when the attachment is attached to the first operational head.

## Description

### FIELD OF THE INVENTION

The subject-matter of the present disclosure relates to personal care appliances, personal care kits.

### BACKGROUND OF THE INVENTION

Some personal care appliances may have more than one operational heads that can be attached to the handle. In addition, some operational heads can have an attachment attached thereto. The operational heads, and also the attachment, may have different functions.

For example, an operational head in the form of an epilator may have a primary function of removing hair. An attachment in the form of a skin stretcher can be attached to the epilator and have a light for selectively emitting a strobe light to improve visibility of the epilator in operation. Another operational head in the form of a near-infrared skin treatment device has a different primary function of emitting light.

Typically, if a single handle is used for each operational head, and optionally the attachment, a different switch is used to control each of the functions. For instance, one switch is used for the primary function of the epilator, a second switch is used for the light of the attachment, and a third switch is used for the light of the near-infrared skin treatment device. However, having multiple switches clutters the handle and can cause confusion for the user.

It is an aim of the subject-matter of the present disclosure to improve on the prior art.

### SUMMARY OF THE INVENTION

According to a first aspect of the present invention, there is provided a personal care appliance, comprising a handle; a first switch on the handle; a second switch on the handle; an illumination device; a detection module configured to detect when a first operational head is attached to the handle, when a second operational head is attached to the handle, and when an attachment is attached to the first operational head; and a controller configured to control a primary function of the first or second operational head in response to actuation of the first switch when the first or second operational head are attached to the handle, to activate the illumination device in response to the detection module detecting the second operational head is attached to the handle or the attachment to the first operational head is attached to the first operational head, and, in response to actuation of the second switch, control a secondary function of the second operational head when the second operational head is attached to the handle, and control a function of the attachment when the attachment is attached to the first operational head.

Changing the function associated with the first switch depending on which operational head is attached to the handle, and using the second switch only for an operational head with a secondary function or for the function of the attachment, reduces clutter on the handle since fewer buttons are required. A reduced number of buttons also reduces the risk of mechanical failures.

In an embodiment, the primary function of the first operational includes a first speed and a second speed.

In an embodiment, the primary function of the second operational head includes turning a light of the second operational head on, and turning the light of the second operational head off.

In an embodiment, the function of the attachment includes turning a light of the attachment on, turning the light of the attachment off, and a frequency of the light emitted by the light of the attachment.

In an embodiment, the detection module is configured to detect that the second operational head comprises a secondary function controllable by the second switch, and the attachment to the first operational head comprises a function controllable by the second switch, and the controller is configured to activate the illumination device in response to detecting the attached second operational head comprises a secondary function controllable by the second switch, or that the attached attachment to the first operational head comprises a function controllable by the second switch.

In an embodiment, the illumination device comprises a light emitting diode, LED.

In an embodiment, the illumination device is configured to illuminate the second switch.

In an embodiment, the first switch and/or the second switch is a mechanical operated switch controlled by a respective actuator.

In an embodiment, the actuator is a push button.

In an embodiment, the push button comprises a pillar portion and a button portion, the pillar portion forming a light guide to guide light from the illumination device to outside the appliance, thereby illuminating the second switch.

In an embodiment, the first operational head is an epilator.

In an embodiment, the attachment is a skin stretching device.

In an embodiment the second operational head is a near-infrared skin treatment device.

According to an aspect of the present invention, there is provided a personal care kit comprising: the personal care appliance of any preceding aspect or embodiment; the first operational head; the second operational head; and the attachment.

These and other aspects of the present invention will be apparent from and elucidated with reference to the embodiment(s) described hereinafter.

### BRIEF DESCRIPTION OF THE DRAWINGS

The embodiments of the present inventions may be best understood with reference to the accompanying figures, in which:
Fig. 1 shows a schematic diagram of a personal care appliance according to one or more embodiments;
Fig. 2 shows a top view of a push button from the personal care appliance of Fig. 1;
Fig. 3 shows a cross-sectional view of the push button from Fig. 2 used to actuate a first switch;
Fig. 4 shows a similar view to Fig. 2 of another push button; and
Fig. 5 shows a similar view to Fig. 3 of another push button.

### DETAILED DESCRIPTION OF THE EMBODIMENTS

At least some of the example embodiments described herein may be constructed, partially or wholly, using dedicated special-purpose hardware. Terms such as 'component', 'module' or 'unit' used herein may include, but are not limited to, a hardware device, such as circuitry in the form of discrete or integrated components, a Field Programmable Gate Array (FPGA) or Application Specific Integrated Circuit (ASIC), which performs certain tasks or provides the associated functionality. In some embodiments, the described elements may be configured to reside on a tangible, persistent, addressable storage medium and may be configured to execute on one or more processors. These functional elements may in some embodiments include, by way of example, components, such as software components, object-oriented software components, class components and task components, processes, functions, attributes, procedures, subroutines, segments of program code, drivers, firmware, microcode, circuitry, data, databases, data structures, tables, arrays, and variables. Although the example embodiments have been described with reference to the components, modules and units discussed herein, such functional elements may be combined into fewer elements or separated into additional elements. Various combinations of optional features have been described herein, and it will be appreciated that described features may be combined in any suitable combination. In particular, the features of any one example embodiment may be combined with features of any other embodiment, as appropriate, except where such combinations are mutually exclusive. Throughout this specification, the term "comprising" or "comprises" means including the component(s) specified but not to the exclusion of the presence of others.

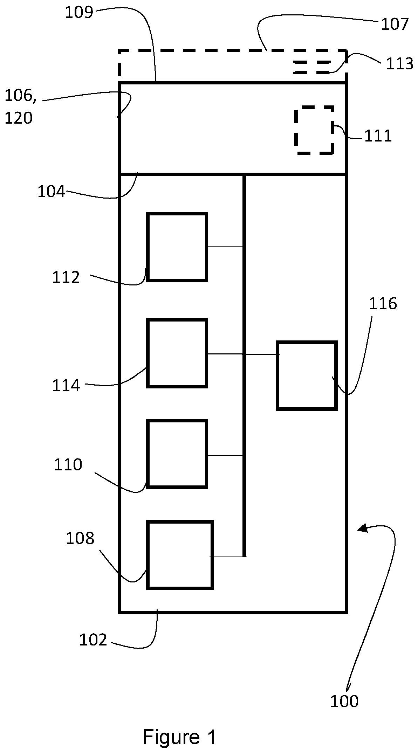

With reference to Fig. 1, a personal care appliance 100 according to one or more embodiments includes a handle 102, an attachment portion 104, a first operational head 106, a second operational head 120, an attachment 107, a power source 108, a controller 110, an actuator 112, a detection module 114, and a user interface 116.

It will be appreciated that the reference signs for the first operational head 106 and the second operational head 120 point to the same feature. This is because the handle 102 is used for multiple different operational heads. The first operational head 106 and the second operational head 120 may be attached to the handle 102 using the attachment portion 104. The first 106 and second operational head 120 may perform different operations, with a different number of functions available for performance of those operations.

The first operational head 106 may be an epilator. The epilator may include a further attachment portion 109. The attachment 107 may attach to the epilator using the further attachment portion 109. The attachment 107 may be a skin stretcher device. The skin stretcher device may include a light 113. The light may be configurable to emit a strobe light pattern, for example. The skin stretcher device is shown as a broken line because it is only attachable to the first operational head 106 and not the second operational head.

The first operational head 106 may have a primary function. The primary function of the first operational head 106 includes setting a speed by selecting a first speed, and selecting a second speed. The first and second speeds are different.

The attachment 107 has a function relating to its light 113. The function may include at least one of turning the light 113 on, turning the light 113 off, and setting a frequency of emitting pulses of light from the light 113. The frequency may be changed to be continually on or emitting a strobe light pattern, for example. The strobe light may be used such that the epilation can be viewed more easily by a user.

The second operational head 120 may be a near-infrared, NIR, skin treatment device. The NIR skin treatment device includes a light 111 for emitting light at an appropriate wavelength, e.g. NIR. The light penetrates into skin of the user to firm up the skin. The light 111 is shown as a broken line since it is not part of the epilator.

The second operational head 120 may have a primary function including at least one of turning on the light 111, and turning off the light 111.

The power source 108 may be a battery, e.g. a secondary, or rechargeable, battery.

The controller 110 may include storage and one or more processors. The storage includes electronic data in the form of instructions. The instructions, when executed by the one or more processors, may cause the one or more processors to perform the computer-implemented methods described herein. In this way, the storage may be non-transitory computer readable media. The instructions may also be provided as transitory computer-readable media when provided as a download to be stored on the storage. Conversely, the personal care appliance may include storage and one or more processors, where the controller 110 is provided as instructions on the storage for execution by the one or more processors.

The actuator 112 is configured to power the first operational head 106 and the second operational head 107. The actuator may be a motor, or the like.

The detection module 114 may be used to determine when the first operational head 107 and/or the second operational head 107 have been attached to the handle 102. The detection module 114 is shown as a function block in Fig. 1. The detection module 114 includes a sensor, storage and one or more processors. The instructions, when executed by the one or more processors, may cause the one or more processors to perform the computer-implemented methods described herein. In this way, the storage may be non-transitory computer readable media. The instructions may also be provided as transitory computer-readable media when provided as a download to be stored on the storage. Conversely, where the personal care appliance includes storage and one or more processors, the detection module 114 may include the sensor and instructions stored on the storage for execution by the one or more processors.

The sensor of the detection module may be an electronic circuit. For example, two electrical contacts are provided which become connected when the first operational head, the second operational head, or the attachment are attached. The detection module can detect which head or attachment is attached by virtue of a level of resistance when the contacts are connected, i.e. when the electronic circuit is closed. This means of detecting the head or attachment that is attached is one illustrative example. Other examples that perform the same purpose may also be used. In this way, the detection module may form an identification module since it is able to identify which head or attachment is attached.

The user interface 116 may be mounted to an exterior surface of the handle 102 and communicatively linked to the controller 110.

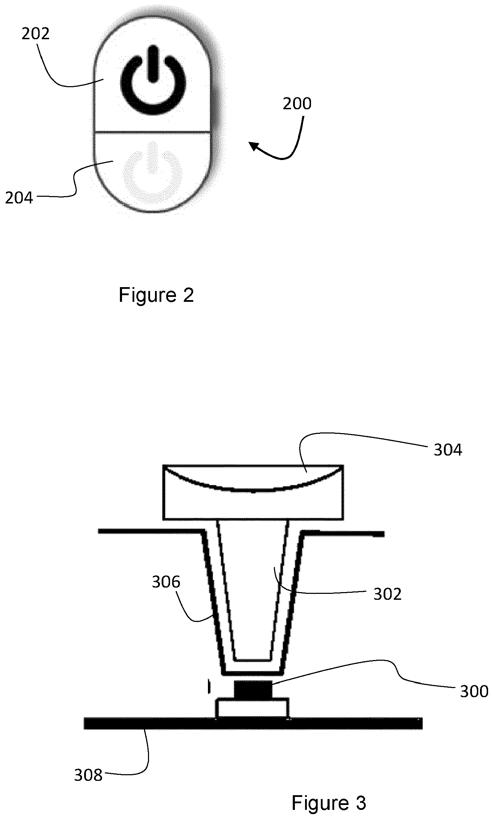

The user interface may include first and second switches, as shown in any of Figs. 2 to 5.

With further reference to Fig. 1, the detection module 114 is configured to detect when the first operational head is attached to the handle, when the second operational head is attached to the handle, and when the attachment is attached to the first operational head. It will be appreciated that only the first operational head 106 or the second operational head 120 may be attached to the handle 102 at one time. Both operational heads cannot be attached to the handle 102 at the same time. Detecting that the first/second operational head 106, 120 or attachment 109 are attached may comprise detecting a primary functionality of the first 106 or second head 120, a functionality of the attachment 109, and a secondary functionality of the second operational head 120. That is, the detection module may suitably determine whether the current operational head and/or attachments comprise additional functionality beyond that of a primary function of the currently attached operational head.

The controller 110 may be configured to control a primary function of the first operational head in response to actuation of a first switch when the first operational head 106 is attached to the handle 102, to control a primary function of the second operational head 120 in response to actuation of the first switch when the second operational head 120 is attached to the handle 102. The controller 110 is similarly configured to control a secondary function of the second operational head 120 or a function of the attachment, in response to actuation of a second switch, when the second operational head 120 or attachment 109 is attached to the handle 102. With reference to Fig. 2, a push button 200 may be provided. The push button 200 may include a first actuator 202 and a second actuator 204. The first and second actuators may be different portions of the push button. For example, the push button 200 may be elongate. The first actuator may be provided at one end of the push button and the second actuator may be provided at the other. In this way, the first actuator and the second actuator may both be a push button. Each actuator is provided to operate a switch. For example, the first actuator 202 may operate the first switch and the second actuator may operate the second switch.

The first switch may be permanently enabled. Suitably, the first switch controls the primary functions of the first operational head 106 - e.g. the hair removal device - and second operational head 120 - e.g., skin treatment device.

The second switch may be enabled only when the second operational head 120 or attachment 109 is attached to the handle 102. The second switch may control secondary functions of the second operational head and the functions of the attachment.

With reference to Fig. 3, the first switch 300 is shown beneath the first actuator of the push button. The second switch may be provided in a similar fashion beath the second actuator.

The first and second switches may be mechanical switches. The push button may comprise a pillar portion 302 and a push button portion 304. A seal 306 may be provided between the actuator and the switch. The first switch 300 and the second switch may be connected to a printed circuit board assembly 308, and thus the controller.

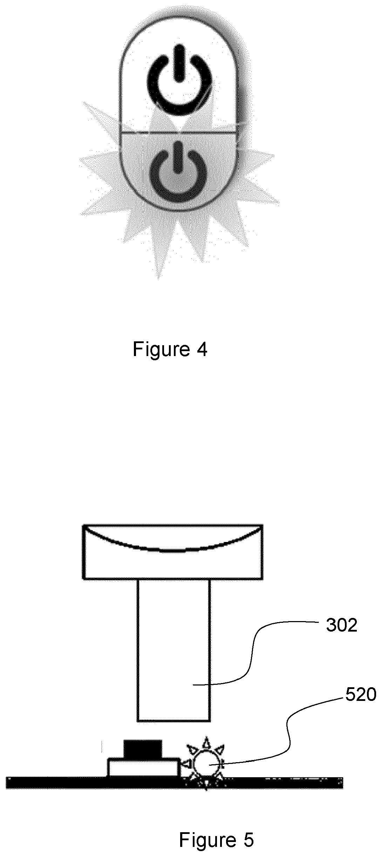

With reference to Figs. 4 and 5, the personal care appliance further comprises an illumination device 520. The illumination device 520 may be located anywhere suitably visible on the personal care appliance 100. For example, the illumination device may be provided on the handle 102. Suitably, the illumination device provides a visual indicator to a user of the appliance 100 that the current operational head / attachment comprises additional functions which may be controlled.

Preferably, the illumination device 520 is configured to illuminate the second switch and or actuator parts thereof (e.g., to illuminate the second actuator 204). Put another way, the illuminator device 520 may be considered to illuminate at least part of a button assembly comprising the second switch. In this way the visual indicator to the user that the current operational head / attachment comprises additional functions naturally guides the user to the button by which those additional functions may be controlled. Suitably, the second button may be designed to be less visible than the first button so that the user is more aware of the first button when only the first button is operational to control a primary function of an operational head. Put another way, the first button may be configured to be more prominent than the second button, which in some examples may be considered a 'hidden' button (hidden to the extent that the user may be less aware of the presence of the second button, not necessarily completely undetectable).

In this example the illumination device 520 comprises a light emitting diode, LED. Here, the pillar portion 302 of the second button forms a light guide to guide light from the illumination device 502 to outside the appliance. Suitably, at least part of the push button portion 304 may be optically transmissive. In this way, the second button may be made to appear to illuminate from inside, which is particularly beneficial to a 'hidden' second button design.

In other embodiments, the first and second switches may be operated by the same actuator. In such cases, a single push button may be used for the first and second actuators by pressing the same part of the push button. However, the controller can distinguish between different functions depending on an amount of time the user presses the push button. For example, a short press may be used for the primary function of the first operational head. A longer pressed may be used for the secondary function of the first operational head. An even longer press may be used for the function of the second operational head.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measured cannot be used to advantage. Any reference signs in the claims should not be construed as limiting the scope.

## Claims

1. A personal care appliance (100), comprising:
a handle (102);
a first switch (300) on the handle;
a second switch on the handle;
an illumination device (520);
a detection module (114) configured to detect when at least one of a first operational head (106) is attached to the handle, a second operational head (120) is attached to the handle, and an attachment (107) is attached to the first operational head; and
a controller (110) configured to:
control a primary function of the first or second operational head in response to actuation of the first switch when the first or second operational head, respectively, is attached to the handle,
activate the illumination device in response to the detection module detecting the second operational head is attached to the handle or the attachment to the first operational head is attached to the first operational head, and
in response to actuation of the second switch, control a secondary function of the second operational head when the second operational head is attached to the handle, and control a function of the attachment when the attachment is attached to the first operational head.

2. The personal care appliance of Claim 1, wherein the primary function of the first operational head includes at least one of a first speed and a second speed.

3. The personal care appliance of Claim 1 or Claim 2, wherein the primary function of the second operational head includes turning a light of the second operational head on, and turning the light of the second operational head off.

4. The personal care appliance of any preceding claim, wherein the function of the attachment includes turning a light of the attachment on, turning the light of the attachment off, and a frequency of pulse of the light emitted by the light of the attachment.

5. The personal care appliance of any preceding claim, wherein the detection module is configured to detect that the second operational head comprises a secondary function controllable by the second switch, and the attachment to the first operational head comprises a function controllable by the second switch, and
the controller is configured to activate the illumination device in response to the detection module detecting the second operational head is attached to the handle and comprises a secondary function controllable by the second switch, or that the attachment to the first operational head is attached to the first operational head and comprises a function controllable by the second switch.

6. The personal care appliance of any preceding claim, wherein the illumination device comprises a light emitting diode, LED.

7. The personal care appliance of any preceding claim, wherein the illumination device is configured to illuminate the second switch.

8. The personal care appliance of any preceding claim, wherein the first switch and/or the second switch is a mechanical operated switch controlled by a respective actuator (202, 204).

9. The personal care appliance of Claim 8, wherein the actuator is a push button (200).

10. The personal care appliance of Claim 9 when dependent upon Claim 7, wherein the push button comprises a pillar portion (302) and a button portion (304), the pillar portion forming a light guide to guide light from the illumination device to outside the appliance.

11. The personal care appliance of any preceding claim, wherein the first operational head is an epilator.

12. The personal are appliance of Claim 11, wherein the attachment is a skin stretching device.

13. The personal care appliance of any preceding claim, wherein the second operational head is a near-infrared skin treatment device.

14. A personal care kit comprising:
the personal care appliance of any preceding claim;
the first operational head;
the second operational head; and
the attachment.
